Europäisches Patentamt

European Patent Office

Office européen des brevets

(19)

(11) Veröffentlichungsnummer : **0 111 777
B1**

(12) **EUROPÄISCHE PATENTSCHRIFT**

(45) Veröffentlichungstag der Patentschrift : .
04.03.87

(21) Anmeldenummer : 83111816.1

(22) Anmeldetag : 25.11.83

(51) Int. Cl.⁴ : **C 07 K 3/18, A 61 K 35/16,
A 61 K 37/02**

(54) **Gerinnungsaktive Plasmaproteinlösung, Verfahren zu ihrer Herstellung und ihre Verwendung zur Behandlung von Störungen des Hämostasesystems.**

(30) Priorität : 21.12.82 DE 3247150

(43) Veröffentlichungstag der Anmeldung :
27.06.84 Patentblatt 84/26

(45) Bekanntmachung des Hinweises auf die Patenterteilung : 04.03.87 Patentblatt 87/10

(84) Benannte Vertragsstaaten :
AT BE CH DE FR GB IT LI LU NL SE

(56) Entgegenhaltungen :
EP-A- 0 014 333
FR-A- 2 293 943
FR-M- 7 395
US-A- 3 998 946

(73) Patentinhaber : Biotest-Serum-Institut GmbH
Flughafenstrasse 4
D-6000 Frankfurt-Niederrad (DE)

(72) Erfinder : Stephan, Wolfgang, Dr. Dipl.-Chem.
Philipp-Holzmann-Strasse 84
D-6072 Dreieich (DE)
Erfinder : Kotitschke, Ronald, Dr. Dipl.-Chem.
Kleiststrasse 23
D-6072 Dreieich (DE)

(74) Vertreter : Wolff, Hans Joachim, Dr.jur. Dipl.-Chem. et al
Beil, Wolff & Beil Rechtsanwälte Postfach 80 01 40
Adelonstrasse 58
D-6230 Frankfurt am Main 80 (DE)

## Beschreibung

Die Erfindung betrifft die in den Patentansprüchen beschriebene gerinnungsaktive Plasmaproteinlösung, ein Verfahren zur Herstellung dieser Lösung und eine pharmazeutische Zubereitung zur intravenösen Behandlung komplexer Störungen des Hämostasesystems, die diese Plasmaproteinlösung enthält.

Die Therapie von Patienten mit Hämostasestörungen mit Blut und Blutkomponenten ist in zahlreichen Publikationen beschrieben (z. B. A.L. Bloom und D.P. Thomas : Haemostasis and Thrombosis, Churchill Livingstone ; Edingburgh, London, Melbourne und New York 1981, S. 472-490 ; K. Lechner : Rationelle Substitutionstherapie bei Gerinnungsstörungen, Infusionstherapie 4 : S. 190-194 (5/1980)). Die intravasalen Gerinnungsstörungen bei den verschiedensten Erkrankungen sind so vielfältig, daß bis heute keine einheitliche Lehrmeinung über die besten Behandlungsmethoden besteht. Dies liegt vor allem daran, daß entsprechende klinische Studien wegen der Vielfat der unterschiedlichen Syndrome praktisch unmöglich sind. In der Praxis der Klinik hat sich die Blutkomponententherapie als erfolgreich erwiesen. Die gebräuchlichsten Mittel dieser Art sind außer dem Frischblut das Frischplasma oder « fresh frozen » Plasma, das gefriergetrocknete Frischplasma, Plättchen-Konzentrate, Kryopräzipitat und die mehr oder weniger hochgereinigten Gerinnungsfaktorenkonzentrate wie das Fibrinogen, das antihämophile Globulin A (= AHG = Faktor VIII) und die Konzentrate, die den Gerinnungsfaktor IX, gegebenenfalls zusammen mit den Faktoren II, VII und X enthalten (= PPSB-Konzentrate).

Die Problematik der Entscheidung über den Einsatz bestimmter Komponenten des Blutes oder auch bestimmter Inhibitoren, wie z. B. Heparin, wird bei dem Krankheitsbild der Verbrauchskoagulopathie deutlich. Die Verbrauchskoagulopathie (= disseminierte oder generalisierte intravaskuläre Gerinnung) ist eine erworbene Blutgerinnungsstörung, die durch einen vermehrten Umsatz plasmatischer Gerinnungsfaktoren und Thrombozyten aufgrund einer generalisierten intravaskulären Gerinnung charakterisiert ist (H.G. Lasch u.a. : Verbrauchskoagulopathien (Pathogenese und Therapie), Folia haemat. (N.F.) 6, S. 325-330 (1961)). Grunderkrankungen, die mit einer Verbrauchskuagulopathie verbunden sein können, sind z. B. ein bakterieller Schock, Verbrennungen, Karzinome, Leukämie, Fruchtwasserembolie, Fettembolie, Acidose, Hypoxämie, Bakteriämie, Viruserkrankungen, Organtransplantationen oder ein anaphylaktischer Schock.

Von E. Lechler u. a. wurde in « Therapie bei Verbrauchs koagulopathie » (Deutsche Medizinische Wochenschrift 100 : Nr. 1, S. 24-25 (1975)) darauf hingewiesen, daß die Verbrauchskoagulopathie zur hämorrhagischen Diathese und durch die Fibrinpräzipitation zur Störung der Mikrozirkulation mit Organnekrosen führt. Die Therapie sollte den intravaskulären Gerinnungsvorgang unterbrechen, und zwar einerseits durch Behandlung der Grundkrankheit bzw. des Pathomechanismus, der die intravaskuläre Gerinnung hervorruft, und andererseits durch Anwendung gerinnungshemmender Mittel. In dieser Arbeit wurde zur Behandlung der Verbrauchskoagulopathie die Heparintherapie empfohlen. Demgegenüber war jedoch G. Wolff in « Fresh frozen plasma : effects and side effects », Bibliotheca haemat., Bd. 46, S. 189-206 (1980) der Meinung, daß gefrorenes Frischplasma ein extrem wirksames Mittel zur Behandlung posttraumatischer Verbrauchskoagulopathien sei, während die Behandlung mit Heparin keineswegs harmlos und deren Basis keinesfalls auf weltweiter Ebene als schlüssig anerkannt sei. Durch Heparin würde nur eine Gerinnungsstörung durch eine andere iatrogene ersetzt.

H. Harke und S. Rahman haben in « Haemostatic Disorders in Massive Transfusion », Bibliotheca haemat. Nr. 46, S. 179-188 (1980) anhand von verschiedenen Zitaten gezeigt, daß die Situation der Patienten durch die Applikation bestimmter Blutkomponenten häufig nicht verbessert, sondern unter Umständen sogar verschlechtert werden kann. So führten Sie an, daß nach Massivtransfusionen diffuse Blutungsneigungen zu den gefürchtetsten postoperativen Komplikationen gehören und diese hämorrhagische Tendenz hauptsächlich auf Störungen des plasmatischen und/oder thrombozytären Gerinnungssystems basiert. Auch sollten charakteristische Änderungen im plasmatischen und thrombozytären System nach Massivtransfusionen die bedeutende Rolle des hämorrhagischen Stocks beim Entstehen von Gerinnungsstörungen zeigen. Ferner schiene das Risiko der Mikroembolisation nach Massivtransfusion bei mehr oder minder großer Schädigung der Organperfusion unvermeidbar und daher sollte der hohen Letalität nach Massivtransfusionen größere Aufmerksamkeit unter dem Aspekt der Organinsuffizienz gewidmet werden, die in gelagertem Blut durch Mikroembolisation und Mikroaggregatbildung auftritt.

Eine Komponente, die in hohem Maß mit zur Verschlechterung der klinischen Situation führen kann, ist das Fibrinogen in den den Patienten zugeführten Plasmen, seien sie nun out dated oder fresh frozen, selbst wenn diese nach den neuesten technischen Erkenntnissen hergestellt werden (vgl. K. Koerner u. a. : « Das tiefgefrorene Frischplasma in der Blutkomponententherapie : Herstellung-Qualitätskontrolle-Indikation », Infusionstherapie 8, S. 253-258 (5/1981)). Aus den zitierten Arbeiten geht hervor, daß gerade die Fibrinpräzipitation zur Störung der Mikrozirkulation mit Organnekrosen führt.

In den DE-PSen 1 617 319 und 1 617 335 wurden bereits Verfahren zur Herstellung von lipoproteinfreien, stabilen und sterilen Seren beschrieben, in denen Blutserum oder Blutplasma mit 250 bis 500 mg kolloidaler Kieselsäure je g Gesamtprotein bei Temperaturen bis zu 50 °C adsorbiert und nach Abtrennung der Kieselsäure UV-bestrahlt und steril filtriert wurde. Die UV-Bestrahlung wurde unter Normalbedingungen, d. h. in Luft, durchgeführt.

Aus der EP-PS 14 333 ist ferner ein Verfahren zur Herstellung mehrerer therapeutisch anwendbarer Plasmaproteinpräparate bekannt, bei dem stabilisiertes Blutplasma an 50 bis 400 mg kolloidaler Kieselsäure pro g Plasmaprotein adsorbiert wurde, um das Fibrinogen zu gewinnen, und die als Zwischenprodukt erhaltene, fibrinogenfreie Plasmaproteinlösung dann weiter aufgearbeitet wurde, um neben anderen Präparaten eine Lösung von lagerstabilen Serumproteinen zu gewinnen. Die als Zwischenprodukt erhaltene Plasmaproteinlösung enthielt auch noch einige Gerinnungsfaktoren. Als Ausgangsmaterial konnte ein mit β-Propiolacton und UV-Bestrahlung (in Luft) behandeltes Citratplasma verwendet werden.

Der Erfindung liegt die Aufgabe zugrunde, eine Plasmaproteinlösung bereitzustellen, deren Fibrinogengehalt wesentlich verringert ist, die jedoch die wichtigen labilen Gerinnungsfaktoren V und VIII und Inhibitoren, wie das Antithrombin III, in unveränderter Form enthält, und die zur Behandlung komplexer Störungen des Hämostasesystems geeignet ist.

Diese Aufgabe wird erfindungsgemäß durch eine gerinnungsaktive Plasmaproteinlösung mit verringertem Fibrinogengehalt, die die wichtigen labilen Gerinnungsfaktoren V und VIII und Inhibitoren, wie das Antithrombin III, in unveränderter Form und alle Gerinnungsfaktoren mit Ausnahme des Fibrinogens und der oberflächenaktiven Faktoren XI und XII in normaler Konzentration enthält und sich nach Gefriertocknung zu einer klaren partikelfreien Lösung rekonstituieren läßt, erhältlich durch mindestens einmalige Adsorption von stabilisiertem Humanplasma mit 20 bis 40 mg kolloidaler Kieselsäure pro g Plasmaprotein und anschließende Trennung des Adsorbens vom Adsorbat, gelöst.

Überraschenderweise wurde gefunden, daß durch eine Herabsetzung der Menge an kolloidaler Kieselsäure auf 20 bis 40 mg pro g Plasmaprotein bei der Adsorption von Humanplasma an kolloidaler Kieselsäure einerseits der Fibrinogengehalt ausreichend um mindestens 50 % verringert, andererseits der Gehalt an den Gerinnungsfaktoren V und VIII ausreichend erhalten werden kann, wobei die Adsorption gegebenenfalls zwecks weiterer Verringerung des Fibrinogengehaltes mehrmals, insbesondere zweimal, durchgeführt werden kann.

Die nachstehende Tabelle I zeigt die Abhängigkeit der Konzentration der Gerinnungsfaktoren im Produkt von der bei der Adsorption verwendeten Menge an kolloidaler Kieselsäure und somit einen Vergleich zwischen der Adsorption mit kolloidaler Kieselsäure gemäß Stufe 1 des Verfahrens der EP-PS 14 333 und der Adsorption gemäß vorliegender Erfindung.

Tabelle I

Abhängigkeit der Konzentration der Gerinnungsfaktoren im Produkt von der bei der Adsorption verwendeten Menge kolloidaler Kieselsäure.

| | Konzentration der Gerinnungsfaktoren in % der Norm | | |
|---|---|---|---|
| | Ausgangsmaterial (Citratplasma) | nach Kieselsäureadsorption mit | |
| | | 250 mg/g [a] | 2x40 mg/g [b] |
| Faktor II | 100 | 95 | 100 |
| Faktor V | 100 | 10 | 95 |
| Faktor VII | 100 | 92 | 100 |
| Faktor VIII | 100 | 20 | 90 |
| Faktor IX | 100 | 98 | 100 |
| Faktor X | 100 | 95 | 100 |
| Faktor XI | 100 | 0 | 10 |
| Faktor XII | 100 | 0 | 10 |
| Antithrombin III | 100 | 98 | 100 |
| Fibrinogen (mg/100 ml) | 300 | 0 | 30 |

[a] : einmalige Adsorption an 250 mg Kieselsäure pro g Protein gemäß EP-PS 14 333
[b] : zwei aufeinanderfolgende Adsorptionen an jeweils 40 mg Kieselsäure pro g Protein.

Wie in Tabelle I gezeigt wird, enthält die erfindungsgemäße Plasmaproteinlösung, die aus Plasma durch Adsorption mit einer Menge an kolloidaler Kieselsäure adsorbiert wird, die unterhalb der nach der EP-PS 14 333 und nach den DE-PSen 1 617 319 und 1 617 335 verwendeten Mengen liegt, alle Gerinnungsfaktoren mit Ausnahme des erfindungsgemäß aus Plasma zu entfernenden Problemfaktors Fibrinogen und der oberflächenaktiven Faktoren XI und XII in normaler Konzentration.

Ein besonders überraschendes Ergebnis bei der erfindungsgemäßen Herstellung von fibrinogenarmen Frischplasma unter Verwendung von kolloidaler Kieselsäure zur Adsorption des Fibrinogens ist der in der nachstehenden Tabelle II dargestellte Befund über die unterschiedliche Adsorbierbarkeit des Faktors VIII an kolloidaler Kieselsäure je nach der Konzentration der Kieselsäure.

Tabelle II

Konzentration von Fibrinogen und Faktor VIII im Plasma in Abhängigkeit von der Konzentration der kolloidalen Kieselsäure bei der Adsorption.

| Kieselsäure mg/g Protein | Faktor VIII Aktivität | % der Norm Antigen | Fibrinogen mg/100 ml |
|---|---|---|---|
| 0 | 100 | 100 | 300 |
| (Ausgangsplasma) | | | |
| 500 [(a)] | 0 | 0 | 0 |
| 1. Adsorption | | | |
| 40 | 95 | 95 | 150 |
| 2. Adsorption | | | |
| 40 | 90 | 90 | 30 |

([a]) gemäß DE-PS 1 617 319

Die Daten der Tabelle II zeigen erneut, daß es erfindungsgemäß bei gezieltem Einsatz bestimmter Mengen kolloidaler Kieselsäure bei der Adsorption von Plasma möglich ist, eine nahezu fibrinogenfreie Plasmaproteinlösung, in der die wesentlichen Gerinnungsfaktoren noch enthalten sind, su gewinnen.

Als Ausgangsmaterial des erfindungsgemäßen Verfahrens wird ein stabilisiertes Humanplasma verwendet, das durch Stabilisieren von Humanblut unter Zugabe von Citratstabilisatoren, Heparin oder anderen bekannten Stabilisatoren oder durch Entzug von Calciumionen mit Hilfe von Ionenaustauschern nach dem in der DE-PS 2 459 291 beschriebenen Verfahren und Abzentrifugieren der Erythrozyten erhalten wurde.

Die für die Adsorption eingesetzte kolloidale Kieselsäure sollte zweckmäßig eine spezifische Oberfläche von 50 bis 400 m$^2$/g aufweisen.

Die Trennung des Adsorbens vom Adsorbat nach der Adsorption mit der kolloidalen Kieselsäure erfolgt vorzugsweise durch Sterilfiltration oder auch durch Ultrafiltration.

Die Vorteile der erfindungsgemäßen Plasmaproteinlösung gegenüber den bekannten Frischplasmen sind vielfältiger Art. Ein Grund für den Zwang, die Frischplasmen in der bisher üblichen Form zu verwenden, bestand darin, daß diese Plasmen sich nicht ohne weiteres in größeren Mengen steril filtrieren ließen. Der Grund für diese Sterilfiltrationsproblematik lag im Fibrinogengehalt der Plasmen. Die erfindungsgemäße Plasmaproteinlösung läßt sich auch unter technischen Bedingungen in großen Maßstab steril filtrieren, da das Fibrinogen aus den Plasmen durch die Adsorption mit de kolloidalen Kieselsäure zu einem wesentlichen Teil entfernt wurde. Ein anderer Grund für die Notwendigkeit der Tiefkühllagerung der Frischplasmen lag in den Lösungsschwierigkeiten fibrinogenhaltiger gefriergetrockneter Plasmen. Das Fibrinogen in diesen Plasmen ließ sich nicht mehr einfach oder häufig nur noch unvollständig in Lösung bringen.

Die erfindungsgemäße Plasmaproteinlösung kann gefriergetrocknet werden und läßt sich nach der Gefriertrocknung zu einer klaren partikelfreien Lösung rekonstituieren.

Dabei kann das Volumen an zugeführtem Wasser, das zum Lösen der gefriergetrockneten Proteine verwendet wird, variiert und so weit reduziert werden, daß die mit weniger Wasser gelöste Proteinlösung bei dann erhöhtem Proteinwert auf entsprechend höhere Werte der Aktivitäten der Gerinnungsfaktoren einstellbar ist.

Die Möglichkeit, verschiedene Frischplasmen zu vereinigen (poolen), um sie zu einer erfindungsgemäßen Plasmaproteinlösung zu verarbeiten, bringt einen weiteren Vorteil gegenüber der Applikation von Einzelspenderplasmen mit sich, der sich auf die Notwendigkeit einer Prüfung der Blutgruppenverträglichkeit des Spenders und Empfängers bezieht. Durch das Poolen von Einzelspenderplasmen zu größeren Pools verdünnen sich die verschiedenen Blutgruppenantikörper des ABO-Systems, so daß die universale Anwendung eines gepoolten Plasmas hinsichtlich des ABO-Systems möglich wird.

Die Tatsache, daß der Faktor XII (Hageman-Faktor) neben dem Fibrinogen durch die kolloidale Kieselsäure bei dem erfindungsgemäßen Verfahren adsorbiert wird, stellt ebenfalls einen deutlichen Vorteil für die Anwendung der erfindungsgemäßen Plasmaproteinlösung gegenüber den herkömmlichen Frischplasmen dar, da der Faktor XII zu den Proteinen gehört, die für den normalen Ablauf einer Hämostase fehlen können (vgl. O.D. Ratnoff : The Surface-mediated Initiation of Blood Coagulation and Related Phenomena in Ogston and Bennett « Haemostasis », John Wiley & Sons, London, New York, Sydney, Toronto, S. 25-55 (1977)), und nach C.J. Paton u. a. : Prekallikrein activator in human albumin, The Lancet, Bd. 3, S. 747 (1981) der Präkallikrein-Aktivator (PKA, Hageman-Faktor-Fragmente) wahrscheinlich für Blutdruckabfall-Zwischenfälle verantwortlich ist, die manchmal bei schneller Infusion von Plasmaprodukten bei der Blutprodukttherapie beobachtet werden.

In einer bevorzugten Ausführungsform der Erfindung wird die erfindungsgemäße Plasmaproteinlösung durch zusätzliche Bestrahlung mit UV-Licht unter einer Inertgasatmosphäre vor oder nach der Adsorption mit kolloidaler Kieselsäure erhalten. Die Bestrahlungsdosis beträgt im allgemeinen 0,5 bis 4 mWatt/cm² × min. Als Inertgasatmosphäre können Stickstoff oder Edelgase, wie Argon oder Krypton, verwendet werden. Vorzugsweise wird eine Stickstoffatmosphäre verwendet.

Eines der wesentlichsten Probleme bei der Transfusion von Frischplasma besteht darin, daß diese Lösungen die Hepatitis übertragen können (L.F. Barker : Posttransfusion Hepatitis ; Epidemiology, Experimental Studies and US Perspective, Bibliotheca Haematologica, Bd. 46, S. 3-14 (1980) ; G.G. Frösner : Nicht-A-nicht-B-Hepatitis, Münch. med. W. 122 Nr. 7, S. 229-230 (1980)). Das Risiko der Hepatitisübertragung durch humane Plasmaproteinlösungen kann bekanntlich durch kombinierte β-Propiolacton- und UV-Behandlung (DE-OS 2 902 158) oder durch Behandlung mit kolloidaler Kieselsäure und UV-Bestrahlung (DE-PSen 1 617 319 und 1 617 335) verhindert werden. Der Nachteil der dort beschriebenen Verfahren besteht jedoch in einem deutlichen bzw. totalen Verlust der Aktivität der plasmatischen Gerinnungsfaktoren. Das erfindungsgemäße Verfahren zur Herstellung eines « Frischplasmas » mit weitgehend verringertem Fibrinogengehalt erlaubt den weitgehenden Erhalt der Aktivität der plasmatischen Gerinnungsfaktoren durch die Anwendung eines Schutzgases bei der UV-Bestrahlung des Plasmas.

Zur Vermeidung des Hepatitisrisikos durch Anwendung von Blutprodukten wurde auch in der EP-PS 14 333 die Behandlung der verwendeten Plasmen mit β-Propiolacton und UV-Bestrahlung beschrieben. Die EP-PS 14 333 zeigt auch den Einfluß dieser Behandlungen auf die Aktivität einiger Gerinnungsfaktoren im Plasma. Für eine Reihe von Gerinnungsfaktoren wurde auch bereits eine unterschiedliche Beeinflussung der Aktivität der Gerinnungsfaktoren durch die einzelnen Behandlungsschritte mit β-Propiolacton und UV beschrieben (R. Kotitschke und W. Stephan : Struktur und Funktion des Fibrinogens, Schattauer-Verlag, Stuttgart-New York 1976, S. 222-228).

Die nachstehende Tabelle III zeigt den Einfluß der Behandlung mit β-Propiolacton und der UV-Bestrahlung mit und ohne Inertgasatmosphäre auf die Aktivität der Faktoren V, VIII und das Antithrombin III in frischem, mit Citrat stabilisiertem und mit kolloidaler Kieselsäure adsorbiertem Plasma.

Tabelle III

Einfluß der Behandlung mit β-Propiolacton und der UV-Bestrahlung auf die Aktivität der Faktoren V, VIII und Antithrombin III in frischem, mit Citrat stabilisiertem und mit kolloidaler Kieselsäure (2 × 40 mg/g Protein) absorbiertem Plasma.

| | Aktivität in % der Norm | | | Phagen pro ml |
|---|---|---|---|---|
| | Faktor V | Faktor VIII | Anti-throm-bin III | |
| a) mit kolloi-daler Kiesel-säure ad-sorbiertes Ausgangs-plasma | 95 | 90 | 100 | $6 \times 10^6$ |

Tabelle 3 (Fortsetzung)

| | Aktivität in % der Norm | | | Phagen pro ml |
|---|---|---|---|---|
| | Faktor V | Faktor VIII | Antithrom- bin III | |
| nach Behand- lung mit ß- Propiolac- ton(0,25%) | 50 | 60 | 60 | $4 \times 10^4$ |
| b) mit kolloida- ler Kiesel- säure adsor- biertes Aus- gangsplasma | 95 | 90 | 100 | $6 \times 10^6$ |
| nach UV-Be- strahlung ( 2 mWatt/ cm² x Min.) in Luft | 65 | 60 | 65 | 0 |
| nach UV-Be- strahlung (2 mWatt/ cm² x Min.) in N$_2$ | 80 | 75 | 80 | 0 |

Die in Tabelle III aufgeführten Ergebnisse über den Einfluß der UV-Bestrahlung auf die Aktivität der empfindlichen Gerinnungsfaktoren V und VIII zeigen, daß überraschenderweise die Aktivitätsminderung dieser Faktoren dadurch deutlich vermindert werden kann, daß die UV-Bestrahlung unter dem Schutz einer Inertgasatmosphäre, insbesondere einer Stickstoffatmosphäre, durchgeführt wird.

Die Ergebnisse von Phagenversuchen, die ebenfalls in Tabelle III dargestellt sind, zeigen, daß die UV-Bestrahlung zur völligen Inaktivierung der Phagen führt.

Ohne wesentlichen Einfluß auf die beschriebenen Ergebnisse über die Adsorptionsbehandlung des Plasma mit kolloidaler Kieselsäure und der UV-Bestrahlung ist die Reihenfolge der Anwendung dieser beiden Verfahrensschritte. Es kann also zunächst der Plasmapool einer UV-Bestrahlung unterworfen werden und anschließend die kolloidale Kieselsäureadsorption erfolgen. Die UV-Bestrahlung kann auch völlig unterbleiben, wenn das Risiko der Hepatitisübertragung durch diagnostische Maßnahmen stark reduziert wurde.

In einer kurzen Übersicht lassen sich die Vorteile der erfindungsgemäßen gerinnungsaktiven Plasmaproteinlösung gegenüber bekannten Lösungen folgendermaßen zusammenfassen :

1. Die Herstellung in technischem Maßstab von großen Plasmapools ist möglich.
2. Eine Behandlung zur Virusinaktivierung ist möglich.
3. Der Erhalt der Aktivität der empfindlichen Plasmafaktoren V und VIII ist möglich.
4. Die Gefriertrocknung ist möglich.

Die Anwendungsvorteile sind im wesentlichen auf folgende Punkte zurückzuführen :

a) Durch die weitgehende Abwesenheit von Fibrinogen wird das Risiko der Verstopfung der Mikrozirkulation durch Fibringerinnsel gemindert.

b) Eine Überprüfung der ABO-Kompatibilität entfällt.

c) Unverträglichkeitsreaktionen durch Präkallikrein werden vermieden.

Die Erfindung wird durch die nachstehenden Beispiele näher erläutert :

Beispiel 1

Herstellung von fibrinogenfreier gerinnungsaktiver Plasmaproteinlösung

Neun Teile Spender-Venenblut wurden zu einem Teil einer 3 %igen Natriumcitrat-Stabilisatorlösung gegeben. Das Blut wurde direkt im Anschluß and die Blutentnahme zentrifugiert. Dadurch wurde die Plasmaproteinlösung von den Erythrozyten abgetrennt, die ihrerseits in physiologischer Kochsalzlösung aufgeschwemmt und dem Spender reinfundiert wurden (Plasmapherese). Das Plasma wurde innerhalb von 2 Stunden nach der Blutentnahme bei —40 °C in einem Kryostaten eingefroren.

Das gefrorene Plasma wurde bei einer Temperatur von + 37 °C aufgetaut. Mehrere Einzelspenderplasmen wurden gepoolt. Das gepoolte Plasma wurde auf + 4 °C abgekühlt und in einer Konzentration von 0,25 g kolloidaler Kieselsäure/100 ml Plasma bzw. 40 mg je g Protein mit kolloidaler Kieselsäure (« Aerosil 380 » der Fa. Degussa) gemischt und für 20 Minuten gerührt. Nach Abtrennen der Kieselsäure durch Zentrifugation wurde eine zweite Adsorption mit kolloidaler Kieselsäure unter gleichen Bedingungen durchgeführt. Die Plasmaproteinlösung wurde anschließend UV-bestrahlt. Dazu wurde der pH-Wert der Lösung auf pH 7,2 eingestellt und die 4 °C kalte Lösung unter einer Stickstoffatmosphäre mit einem UV-Durchfluß-Bestrahlungsgerät in einer Dosierung von 2mWatt/cm² × min UV-bestrahlt.

Anschließend wurde die UV-bestrahlte fibrinogenarme Plasmaproteinlösung in Mengen von jeweils 250 ml über durch Heißluft sterilisierte Membranfilter der Porengröße 0,45 und 0,22 μm in leere, sterile 500 ml-Flaschen abgefüllt.

Die sterilfiltrierte Proteinlösung wurde im Kryostaten bei —40 °C einrolliert (spin freezing) und anschließend gefriergetrocknet.

Durch Lösen des gefriergetrockneten Präparates in destilliertem Wasser auf einen Proteingehalt von 5 g/100 ml wurde eine zur intravenösen Anwendung geeignete gerinnungsaktive Plasmaproteinlösung erhalten, die die in der nachstehenden Tabelle IV aufgeführten Eigenschaften aufwies.

Tabelle IV

Eigenschaften der gerinnungsaktiven Plasmaproteinlösung (Proteingehalt 5,0 g/100 ml)

| | Aktivität in % der Norm |
|---|---|
| Faktor II | 95 |
| Faktor V | 60* |
| Faktor VII | 90 |
| Faktor VIII | 70* |
| Faktor IX | 95 |
| Faktor X | 95 |
| Faktor XI | 10 |
| Faktor XII | 10 |
| Antithrombin III | 80 |
| Fibrinogen (mg/100ml) | 30 |

* Die Unterschiede zu den Daten der Tabelle III sind auf Verluste durch Sterilfiltration, Gefriertrocknung usw. zurückzuführen.

Der Gehalt der in Tabelle IV nicht aufgeführten Proteine, wie z. B. der Immunglobuline und des Albumins, in der hier erhaltenen Proteinlösung entspricht dem von Frischplasma.

## Beispiel 2

Es wurde die Arbeitsweise von Beispiel 1 wiederholt, wobei jedoch anstelle der Natriumcitratlösung für die Stabilisierung des Spender-Venenblutes Heparin als Stabilisator eingesetzt wurde. Das so erhaltene Ausgangsplasma wurde nach der Arbeitsweise von Beispiel 1 behandelt. Es wurde eine heparinhaltige gerinnungsaktive Plasmaproteinlösung erhalten, deren Eigenschaften denen der Tabelle IV entsprachen.

## Beispiel 3

Es wurde die Arbeitsweise von Beispiel 1 wiederholt, wobei jedoch anstelle der Natriumcitratlösung für die Stabilisierung des Spender-Venenblutes ein Ionenaustauscher eingesetzt wurde. Die Herstellung des Ionenaustauscherplasmas erfolgte nach dem in der DE-PS 24 59 291 beschriebenen Verfahren. Das so hergestellte Ionenaustauscherplasma wurde nach der Arbeitsweise von Beispiel 1 behandelt. Es wurde eine gerinnungsaktive Plasmaproteinlösung erhalten, deren Eigenschaften denen der Tabelle IV entsprachen.

## Beispiel 4

Nach der Arbeitsweise von Beispiel 1 wurde ein Plasmapool hergestellt, der mit einem UV-Durchfluß-Bestrahlungsgerät mit 2 mWatt/cm² x min UV-bestrahlt wurde. Das UV-bestrahlte Plasma wurde auf + 4 °C abgekühlt und nach der Arbeitsweise von Beispiel 1 zweimal der Adsorption mit kolloidaler Kieselsäure unterworfen.

Die weitere Aufarbeitung und Gefriertrocknung erfolgte ebenfalls nach der Arbeitsweise von Beispiel 1. Die Eigenschaften der durch Lösen des gefriergetrockneten Präparates erhaltenen Lösung entsprachen denen der Tabelle IV.

## Beispiel 5

Citratplasma wurde auf einen pH-Wert von 7,2 eingestellt, auf + 4°C abgekühlt und nach der Arbeitsweise von Beispiel 1 mit kolloidaler Kieselsäure (« Aerosil 380 ») gerührt. Diese Mischung wurde bei — 80 °C eingefroren. Die eingefrorene Mischung wurde bei + 37 °C aufgetaut und die Kieselsäure durch Zentrifugation abgetrennt. Der Überstand wurde erneut mit kollidaler Kieselsäure (« Aerosil 380 ») unter den obengenannten Bedingungen behandelt und die Mischung erneut bei — 80 °C eingefroren und bei + 37 °C aufgetaut und durch Zentrifugation von der Kieselsäure getrennt. Das doppelt adsorbierte, fibrinogen-arme Plasma wurde sterilfiltriert, bei — 40 °C einrolliert und gefriergetrocknet.

**Patentansprüche** (für die Vertragsstaaten : BE, CH, DE, FR, GB, IT, LI, LU, NL, SE)

1. Gerinnungsaktive Plasmaproteinlösung mit verringertem Fibrinogengehalt, die die wichtigen labilen Gerinnungsfaktoren V und VIII und Inhibitoren, wie das Antithrombin III, in unveränderter Form und alle Gerinnungsfaktoren mit Ausnahme des Fibrinogens und der oberflächenaktiven Faktoren XI und XII in normaler Konzentration enthält und sich nach Gefriertrocknung zu einer klaren partikelfreien Lösung rekonstituieren läßt, erhältlich durch mindestens einmalige Adsorption von stabilisiertem Humanplasma mit 20 bis 40 mg kolloidaler Kieselsäure pro g Plasmaprotein und anschließende Trennung des Adsorbens vom Adsorbat.

2. Plasmaproteinlösung nach Anspruch 1, dadurch gekennzeichnet, daß sie durch zusätzliche Bestrahlung mit UV-Licht unter einer Inertgasatmosphäre vor oder nach der Adsorption mit kolloidaler Kieselsäure erhältlich ist.

3. Plasmaproteinlösung nach einem der Ansprüche 1 oder 2, dadurch gekennzeichnet, daß sie durch zweimalige Adsorption mit jeweils 20 bis 40 mg kolloidaler Kieselsäure pro g Plasmaprotein erhältlich ist.

4. Verfahren zur Herstellung einer gerinnungsaktiven Plasmaproteinlösung nach Anspruch 1, dadurch gekennzeichnet, daß man stabilisiertes Humanplasma mindestens einmal an 20 bis 40 mg kolloidaler Kieselsäure pro g Plasmaprotein adsorbiert und anschließend das Adsorbens vom Adsorbat trennt.

5. Verfahren nach Anspruch 4, dadurch gekennzeichnet, daß man zusätzlich vor oder nach der Adsorption mit kolloidaler Kieselsäure eine Bestrahlung mit UV-Licht unter einer Inertgasatmosphäre durchführt.

6. Verfahren nach einem der Ansprüche 4 oder 5, dadurch gekennzeichnet, daß man das Humanplasma zweimal an jeweils 20 bis 40 mg kolloidaler Kieselsäure pro g Plasmaprotein adsorbiert.

7. Verfahren nach einem der Ansprüche 4 bis 6, dadurch gekennzeichnet, daß man als stabilisiertes Humanplasma ein Plasma verwendet, das aus Humanblut durch Zugabe von Citratstabilisatoren oder Heparin oder Entzug von Calciumionen mit Hilfe von Ionenaustauschern erhalten wurde.

8. Verfahren nach einem der Ansprüche 4 bis 7, dadurch gekennzeichnet, daß man zur Trennung des Adsorbens vom Adsorbat eine Sterilfiltration durchführt.

9. Pharmazeutische Zubereitung zur intravenösen Behandlung komplexer Störungen des Hämostasesystems, gekennzeichnet durch einen Gehalt an einer Plasmaproteinlösung nach einem der Ansprüche 1 bis 8.

10. Zubereitung nach Anspruch 9, dadurch gekennzeichnet, daß sie die Plasmaproteinlösung in gefriergetrocknetem Zustand enthält.

**Patentansprüche** (für den Vertragsstaat AT)

1. Verfahren zur Herstellung einer gerinnungsaktiven Plasmaproteinlösung mit verringertem Fibrinogengehalt, die die wichtigen labilen Gerinnungsfaktoren V und VIII und Inhibitoren, wie das Antithrombin III, in unveränderter Form und alle Gerinnungsfaktoren mit Ausnahme des Fibrinogens und der oberflächenaktiven Faktoren XI und XII in normaler Konzentration enthält und sich nach Gefriertrocknung zu einer klaren partikelfreien Lösung rekonstituieren läßt, dadurch gekennzeichnet, daß man stabilisiertes Humanplasma mindestens einmal an 20 bis 40 mg kolloidaler Kieselsäure pro g Plasmaprotein adsorbiert und anschließend das Adsorbens vom Adsorbat trennt.

2. Verfahren nach Anspruch 1, dadurch gekennzeichnet, daß man zusätzlich vor oder nach der Adsorption mit kolloidaler Kieselsäure eine Bestrahlung mit UV-Licht unter einer Inertgasatmosphäre durchführt.

3. Verfahren nach einem der Ansprüche 1 oder 2, dadurch gekennzeichnet, daß man das Humanplasma zweimal an jeweils 20 bis 40 mg kolloidaler Kieselsäure pro g Plasmaprotein adsorbiert.

4. Verfahren nach einem der Ansprüche 1 bis 3, dadurch gekennzeichnet, daß man als stabilisiertes Humanplasma ein Plasma verwendet, das aus Humanblut durch Zugabe von Citratstabilisatoren oder Heparin oder Entzug von Calciumionen mit Hilfe von Ionenaustauschern erhalten wurde.

5. Verfahren nach einem der Ansprüche 1 bis 4, dadurch gekennzeichnet, daß man zur Trennung des Adsorbens vom Adsorbat eine Sterilfiltration durchführt.

6. Verfahren zur Herstellung einer pharmazeutischen Zubereitung zur intravenösen Behandlung komplexer Störungen des Hämostasesystems, dadurch gekennzeichnet, daß man eine gerinnungsaktive Plasmaproteinlösung nach einem der Ansprüche 1 bis 5 herstellt und die erhaltene Lösung gefriertrocknet.

**Claims** (for the Contracting States : BE, CH, DE, FR, GB, IT, LI, LU, NL, SE)

1. Coagulation-active plasmaprotein solution having a reduced fibrinogen content, containing the important instable coagulation factors V and VIII and inhibitors like antithrombin III in an unchanged form and all coagulation factors except fibrinogen and the surface-active factors XI and XII in normal concentration, and being reconstitutionable after freeze-drying into a clear particle-free solution, obtainable by at least one adsorption of stabilized human plasma with from 20 to 40 mg colloidal silicic acid per g plasmaprotein and subsequent separation of the adsorbent from the adsorbate.

2. Plasmaprotein solution according to claim 1, characterized in being obtainable by an additional irradiation with ultra-violet light in an inert gas atmosphere before or after the adsorption with colloidal silicic acid.

3. Plasmaprotein solution according to one of claims 1 or 2, characterized in being obtainable by a twice adsorption with from 20 to 40 mg each of colloidal silicic acid per g plasmaprotein.

4. Process for the preparation of a coagulation-active plasmaprotein solution according to claim 1, characterized in adsorbing stabilized human plasma at least once on from 20 to 40 mg colloidal silicic acid per g plasmaprotein and subsequently separating the adsorbent from the adsorbate.

5. Process according to claim 4, characterized in additionally carrying out an irradiation with ultra-violet light under an inert gas atmosphere before or after the adsorption with colloidal silicic acid.

6. Process according to one of claims 4 or 5, characterized in twice adsorbing the human plasma on from 20 to 40 g each of colloidal silicic acid per g plasmaprotein.

7. Process according to one of claims 4 to 6, characterized in using as stabilized human plasma a plasma obtained from human blood by adding citrate stabilizers or heparin or by withdrawing calcium ions with the aid of ion exchangers.

8. Process according to one of claims 4 to 7, characterized in carrying out a sterile filtration to separate the adorbent from the adsorbate.

9. Pharmaceutical preparation for the intravenous treatment of complex disorders of the hemostasis system, characterized in containing a plasmaprotein solution according to one of claims 1 to 8.

10. Preparation according to claim 9, characterized in containing the plasmaprotein solution in freeze-dried state.

**Claims** (for the Contracting State AT)

1. Process for the preparation of a coagulation-active plasmaprotein solution having a reduced fibrinogen content, containing the important instable coagulation factors V and VIII and inhibitors like the antithrombin III, in an unchanged form and all coagulation factors except the fibrinogen and the surface-active factors XI and XII in normal concentration, and being reconstitutable after freeze-drying into a clear particle-free solution, characterized in adsorbing stabilized human plasma at least once on from 20 to 40 mg colloidal silicic acid per g plasmaprotein and subsequently separating the adsorbent from the adsorbate.

2. Process according to claim 1, characterized in additionally carrying out an irradiation with ultra-violet light under an inert gas atmosphere before or after the adsorption with colloidal silicic acid.

3. Process according to one of claims 1 or 2, characterized in twice adsorbing the human plasma on from 20 to 40 mg each of colloidal silicic acid per g plasmaprotein.

4. Process according to one of claims 1 to 3, characterized in using as stabilized human plasma a plasma obtained from human blood by adding citrate stabilizers or heparin or by withdrawing calcium ions with the aid of ion exchangers.

5. Process according to one of claims 1 to 4, characterized in carrying out a sterile filtration to separate the adsorbent from the adsorbate.

6. Process for preparing a pharmaceutical preparation for the intravenous treatment of complex disorders of the hemostasis system, characterized in preparing a coagulation-active plasmaprotein solution according to one of claims 1 to 5 and freeze-drying the so obtained solution.


**Revendications** (pour les Etats contractants : BE, CH, DE, FR, GB, IT, LI, LU, NL, SE)

1. Solution de protéines du plasma ayant une activité coagulante à teneur réduite en fibrinogène, qui contient les facteurs de coagulation labiles importants V et VIII et des inhibiteurs, comme l'antithrombine III, sous forme non modifiée et tous les facteurs de la coagulation à l'exception du fibrinogène et des facteurs tensioactifs XI et XII à concentration normale et peut être reconstituée après lyophilisation en une solution claire dépourvue de particules, qui s'obtient par au moins une adsorption de plasma humain stabilisé avec de 20 à 40 mg d'acide silicique colloïdal par g de protéine du plasma puis séparation de l'adsorbant d'avec l'adsorbat.

2. Solution de protéines du plasma selon la revendication 1, caractérisée en ce qu'on l'obtient par une irradiation supplémentaire avec la lumière UV sous une atmosphère de gaz inerte avant ou après l'adsorption avec de l'acide silicique colloïdal.

3. Solution de protéines du plasma selon l'une des revendications 1 et 2, caractérisée en ce qu'on l'obtient par deux adsorptions avec à chaque fois de 20 à 40 mg d'acide silicique colloïdal par g de protéine du plasma.

4. Procédé de préparation d'une solution de protéines du plasma ayant une activité coagulante selon la revendication 1, caractérisé en ce qu'on adsorbe du plasma humain stabilisé au moins une fois sur 20 à 40 mg d'acide silicique colloïdal par g de protéine du plasma puis en ce qu'on sépare l'adsorbant d'avec l'adsorbat.

5. Procédé selon la revendication 4, caractérisé en ce qu'on effectue en outre avant ou après l'adsorption avec l'acide silicique colloïdal une irradiation avec de la lumière UV sous une atmosphère de gaz inerte.

6. Procédé selon l'une des revendications 4 et 5, caractérisé en ce qu'on adsorbe le plasma humain deux fois sur à chaque fois 20 à 40 mg d'acide silicique colloïdal par g de protéine du plasma.

7. Procédé selon l'une des revendications 4 à 6, caractérisé en ce qu'on utilise comme plasma humain stabilisé un plasma que l'on a obtenu à partir de sang humain par addition de stabilisateurs au citrate ou d'héparine ou élimination des ions calcium à l'aide d'échangeurs d'ions.

8. Procédé selon l'une des revendications 4 à 7, caractérisé en ce qu'on effectue une filtration stérile pour séparer l'adsorbant d'avec l'adsorbat.

9. Préparation pharmaceutique pour le traitement intraveineux de troubles complexes du système d'hémostase, caractérisée en ce qu'elle contient une solution de protéines du plasma selon l'une des revendications 1 à 8.

10. Préparation selon la revendication 9, caractérisée en ce qu'elle contient la solution de protéines du plasma à l'état lyophilisé.


**Revendications** (pour l'Etat contractant AT)

1. Procédé de préparation d'une solution de protéines du plasma ayant une activité coagulante et à teneur réduite en fibrinogène, qui contient les facteurs de coagulation labiles importants V et VIII et des inhibiteurs, comme l'antithrombine III, sous forme non modifiée et tous les facteurs de la coagulation à l'exception du fibrinogène et des facteurs tensio-actifs XI et XII à concentration normale et peut être partiellement reconstituée après lyophilisation en une solution claire dépourvue de particules, caractérisé en ce qu'on adsorbe du plasma humain stabilisé au moins une fois sur 20 à 40 mg d'acide silicique colloïdal par g de protéine du plasma puis en ce qu'on sépare l'adsorbant d'avec l'adsorbat.

2. Procédé selon la revendication 1, caractérisé en ce qu'en outre avant ou après l'adsorption avec l'acide silicique colloïdal on effectue une irradiation avec la lumière UV sous une atmosphère de gaz inerte.

3. Procédé selon l'une des revendications 1 et 2, caractérisé en ce qu'on adsorbe le plasma humain deux fois sur à chaque fois 20 à 40 mg d'acide silicique colloïdal par g de protéine du plasma.

4. Procédé selon l'une des revendications 1 à 3, caractérisé en ce qu'on utilise comme plasma humain stabilisé un plasma que l'on a obtenu à partir de sang humain par addition de stabilisateurs au citrate ou d'héparine ou élimination d'ions calcium à l'aide d'échangeurs d'ions.

5. Procédé selon l'une des revendications 1 à 4, caractérisé en ce qu'on effectue une filtration stérile pour séparer l'adsorbant d'avec l'adsorbat.

6. Procédé de préparation d'une préparation pharmaceutique pour le traitement intraveineux de troubles complexes du système d'hémostase, caractérisé en ce qu'on prépare une solution de protéines du plasma ayant une action coagulante selon l'une des revendications 1 à 5 et en ce qu'on lyophilise la solution obtenue.